# EUROPÄISCHE PATENTSCHRIFT

(11) **EP 0 801 935 B1**
(45) Veröffentlichungstag und Bekanntmachung des Hinweises auf die Patenterteilung: **03.07.2002**
(21) Anmeldenummer: 97105338.4
(22) Anmeldetag: 29.03.1997
(51) Int. Cl.: A61F 2/34

(54) **Prothetische Gelenkpfanne**
Acetabular cup prosthesis
Prothèse de coque acétabulaire

(30) Priorität: 20.04.1996 DE 19615786
(43) Veröffentlichungstag der Anmeldung: 22.10.1997
(73) Patentinhaber: CERASIV GmbH INNOVATIVES KERAMIK-ENGINEERING, D-73207 Plochingen (DE)
(72) Erfinder: Kälberer, Hartmut, 73779 Deizisau (DE); Brehm, Peter, 91085 Weisendorf (DE); Hennig, F.F., Prof. Dr. med., 91054 Erlangen (DE)
(74) Vertreter: Scherzberg, Andreas, Dr.

(56) Entgegenhaltungen:
- EP-A- 0 351 545
- DE-A- 4 239 263
- US-A- 4 159 544

## Beschreibung

Die Erfindung bezieht sich auf eine prothetische Gelenkpfanne zur Aufnahme eines Kugelkopfes.

Bei einem prothetischen Gelenk werden zur Aufnahme eines Kugelkopfes implantierbare Gelenkpfannen verwendet. Die Gelenkpfanne besteht im wesentlichen aus einer Außenschale und aus einer darin eingesetzten Innenschale.

Aus EP-A-0 234 811 ist eine Gelenkpfanne bekannt, deren Außenschale aus Metall und deren Innenschale aus Kunststoff besteht. Bei großen Belastungen kann der eingesetzte Kugelkopf mit hohen Drücken die Kunststoffinnenschale plastisch verformen, wodurch die Gelenkgeometrie verändert wird. Ferner entsteht Abrieb der Kunststoffinnenschale, der in das angrenzende Gewebe gelangen kann.

Aus EP-A-0 315 795 ist eine Gelenkpfanne bekannt, deren Außenschale aus Kunststoff und deren Innenschale aus Metall besteht. Wegen der geringen Festigkeit des Kunststoffes kann es auch hier zu Lockerungen des Gelenkpfannensitzes im Knochen kommen. Durch die Reibung der Gelenkkugel in der Metallinnenschale werden Metallpartikel freigesetzt, die in das umliegende Gewebe eindringen können.

Die Befestigung der Innenschale in der Außenschale erfordert besondere konstruktive Maßnahmen, wie die Verwendung von Sicherungsringen, Verklebung oder Verklemmung. Bei starker mechanischer Beanspruchung wird die Substanz der Innenschalen reduziert, weshalb eine regelmäßige Kontrolle und ggf. ein Austausch der Innenschale erforderlich ist.

Die gattungsgemäße EP-A-0 351 545 zeigt eine prothetische Gelenkpfanne zur Aufnahme eines Kugelkopfes, wobei die Gelenkpfanne von einem einzigen einstückigen Schalenkörper gebildet ist, der Schalenkörper aus Keramik besteht, am Öffnungsrand des Schalenkörpers ein entfernbarer Sicherungsring zum Sichern des eingesteckten Kugelkopfes vorgesehen ist und der Innendurchmesser des Sicherungsrings kleiner als der Durchmesser des Kugelkopfes ist.

Aufgabe der Erfindung ist es, eine verbesserte prothetische Gelenkpfanne zu schaffen.

Die Aufgabe wird erfindungsgemäß durch die Merkmale des Anspruchs 1 gelöst.

Die Gelenkpfanne wird von einem einzigen einstückigen Schalenkörper gebildet. Der Schalenkörper dient an seiner Innenseite der Aufnahme des Kugelkopfes, während seine Außenseite in den Knochen implantiert wird. Die Konstruktion und Herstellung dieser Gelenkpfanne ist u. a. durch den Wegfall von Verbindungselementen stark vereinfacht.

Der Schalenkörper besteht aus Keramik, wodurch insbesondere bei Verwendung eines keramischen Kugelkopfes eine sehr exakte spalt- und spielfreie Lagerung des Kugelkopfes ermöglicht wird. Dies ändert sich wegen der gleichen Ausdehnungskoeffizienten des keramischem Kugelkopfes und des keramischen Schalenkörpers auch nicht bei Temperaturänderungen.

Die Keramik ist aufgrund ihrer Härte sehr verschleißarm, so daß kein nennenswerter Abrieb auftritt, der in benachbartes Gewebe eindringen könnte. Ferner ist die Keramik sehr verschleißfest, was bei Verwendung eines Keramikkugelkopfes bewirken kann, daß auch bei stark beanspruchten Gelenkprothesen, beispielsweise Hüftprothesen, eine sehr lange Lebensdauer der Gelenkpfanne erreicht und ein Auswechseln der Gelenkpfanne vermieden wird.

Am Öffnungsrand des Schalenkörpers ist in einer umlaufenden Ausnehmung ein entfernbarer Sicherungsring zum Sichern des eingesteckten Kugelkopfes vorgesehen. Der Innendurchmesser des Sicherungsrings ist kleiner als der Durchmesser des Kugelkopfes. Der Sicherungsring hält den Kugelkopf in dem Schalenkörper fest. Dadurch wird verhindert, daß der Kugelkopf kurzzeitig aus der Gelenkpfanne gezogen wird, bzw. vollständig austritt. Durch Vermeidung auch kleinerer Austritte des Kugelkopfes aus dem Schalenkörper werden Beschädigungen und Verschleiß vermieden, die beim Zurückschnellen des Kugelkopfes in die Gelenkpfanne auftreten können. Der Sicherungsring erhöht dadurch die Lebensdauer der Gelenkpfanne.

Der Sicherungsring kann an seiner Außenseite einen umlaufenden Steg aufweisen, der mit einer umlaufenden Nut an der Innenseite des Schalenkörpers zusammengreift. Durch Einrasten des Steges in die Nut wird der Sicherungsring an dem Schalenkörper mit einfachen Mitteln befestigt.

Der Sicherungsring weist vorzugsweise eine geringere Härte auf, als das Kugelkopfmaterial und besteht vorzugsweise aus Polyethylen. Bei Zugbelastungen des Gelenks können die Ausrückbewegungen des Kugelkopfes im Bereich des Sicherungsringes große Ausrückkräfte bewirken, die von dem Sicherungsring aufgefangen werden müssen. Durch Wahl eines elastischen und weichen Sicherungsring-Materials wird der Kugelkopf in seiner Ausrückbewegung elastisch abgebremst und werden Kratzer und Beschädigungen des Kugelkopfes durch den Sicherungsring vermieden.

Der Sicherungsring weist erfindungsgemäß an seiner Außenseite eine umlaufende Nut auf, die die axiale Verformbarkeit des Sicherungsringes erhöht und dadurch dafür sorgt, daß der austretende Kugelkopf elastischer abgebremst wird.

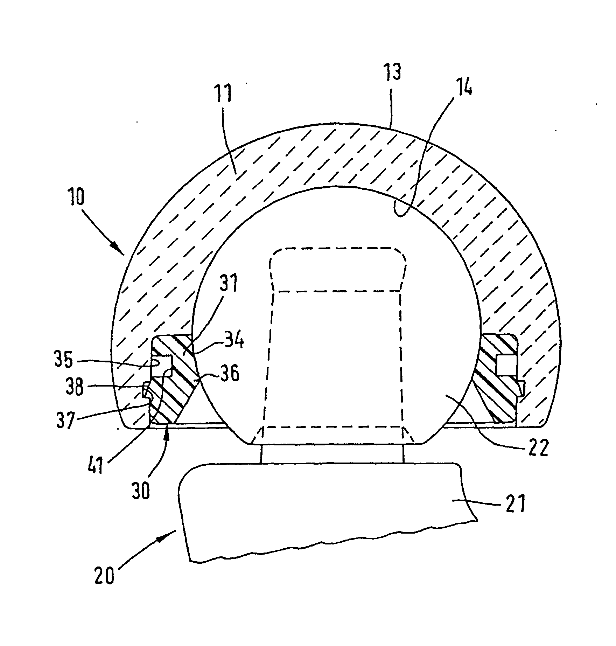

Im folgenden wird unter Bezugnahme auf die Zeichnung ein Ausführungsbeispiel der Erfindung näher erläutert.

Die Figur zeigt einen Längsschnitt eines einstückigen Schalenkörpers mit eingesetztem Sicherungsring.

Eine prothetische Gelenkpfanne 10 wird von einem einstückigen Schalenkörper 11 aus Keramik und einem Sicherungsring 30 gebildet. Die Außenseitenwand 13 und die Innenseitenwand 14 des Schalenkörpers 11 sind jeweils kugelförmig ausgebildet.

In die Gelenkpfanne 10 ist eine Kugelkopfprothese 20 eingesteckt, die aus einem kugelförmigen Kugelkopf 22 und dem Prothesenschaft 21 besteht. Der Außendurchmesser des Kugelkopfes 22 ist annähernd gleich dem Innendurchmesser der kugelförmigen Innenseitenwand 14 der Gelenkpfanne 10, so daß der Kugelkopf 22 in dem Schalenkörper 11 spalt- und spielfrei gelagert ist. Dadurch wird eine großflächige Übertragung von Druckkräften zwischen der Gelenkpfanne 10 und der Kugelkopfprothese 20 gewährleistet.

Im Bereich des Öffnungsrandes des Schalenkörpers 11 ist der Sicherungsring 30 eingesetzt, der von einem ringartigen profilierten Ringkörper 31 aus Polyethylen gebildet wird. Der Ringkörper 31 ist in eine umlaufende Ausnehmung 35 am Öffnungsrand des Schalenkörpers 11 eingesetzt, so daß der Ringkörper 21 nicht in die kugelförmige Ausnehmung des Schalenkörpers 11 hineinragt. Die dem Schalenkörperinnenraum zugewandte Innenumfangsseite 34 des Ringkörpers 31 ist im Querschnitt konkav gewölbt und setzt die halbkugelförmige Innenseitenwand 14 des Schalenkörpers 11 zur Öffnungsseite hin fort. An der öffnungsseitigen Kante 36 der Innenumfangsseite 34 ist der Innenumfang bzw. Durchmesser kleiner als der Außenumfang bzw. der Durchmesser des Kugelkopfes 22. Bei eingesetztem Sicherungsring 20 kann der Kugelkopf 22 daher nicht aus dem Schalenkörper 11 heraustreten.

Der Schalenkörper 11 weist in der Ausnehmung 35 eine radiale umlaufende Nut 37 auf, in die ein radialer umlaufender Steg 38 des Ringkörpers 31 eingreift. Dadurch wird der Ringkörper 31 an dem Schalenkörper gegen die Ausrückkräfte des Kugelkopfes 22 gesichert. Dennoch kann der Sicherungsring 30 ggf. mit einfachen Mitteln entfernt werden, um die Kugelkopfprothese 20 von der Gelenkpfanne 10 zu trennen.

An der Außenseite des Ringkörpers 31 ist eine radiale umlaufende Nut 41 eingelassen. Sie bewirkt bessere Verformungseigenschaften des Ringkörpers 31 in axialer Richtung, wodurch die Federwirkung des Sicherungsrings 30 verbessert wird. Bei einer Austrittsbewegung des Kugelkopfes 22 gibt der Ringkörper 31 federnd nach, wodurch die Auswärtsbewegung des Kugelkopfes 22 sanft abgebremst wird.

Der Sicherungsring kann auch aus anderen Materialien bestehen, die jedoch weicher als die Schalenkörperkeramik und möglichst elastisch sein sollten.

Mit dem einstückigen Schalenkörper aus Keramik ist eine Gelenkpfanne geschaffen, die eine hohe Gelenkpräzision und lange Lebensdauer bietet.

## Patentansprüche

1. Prothetische Gelenkpfanne zur Aufnahme eines Kugelkopfes (22), wobei die Gelenkpfanne von einem einzigen einstückigen Schalenkörper (11) gebildet ist, der Schalenkörper aus Keramik besteht, am Öffnungsrand des Schalenkörpers (11) ein entfernbarer Sicherungsring (30) zum Sichern des eingesteckten Kugelkopfes (22) vorgesehen ist und der Innendurchmesser des Sicherungsrings (30) kleiner als der Durchmesser des Kugelkopfes (22) ist, **dadurch gekennzeichnet, dass** der Sicherungsring (30) an seiner Außenseite eine umlaufende Nut (41) aufweist.

2. Prothetische Gelenkpfanne nach Anspruch 1, **dadurch gekennzeichnet, dass** der Sicherungsring (30) in einer umlaufenden Ausnehmung (35) angeordnet ist.

3. Prothetische Gelenkpfanne nach Anspruch 1 oder 2, **dadurch gekennzeichnet, dass** der Sicherungring (30) an seiner Außenseite einen umlaufenden Steg (38) aufweist, der mit einer umlaufenden Nut (37) an der Innenseite des Schalenkörpers (11) zusammengreift.

4. Prothetische Gelenkpfanne nach einem der Ansprüche 1 bis 3, **dadurch gekennzeichnet, dass** der Sicherungsring (30) aus einem Material besteht, das eine geringere Härte als das Kugelkopfmaterial aufweist, und vorzugsweise aus Polyethylen besteht.

## Claims

1. A prosthetic joint socket for receiving a ball head (22), the joint socket being formed by a single, integrally formed shell body (11), the shell body being made of ceramic material, a removable securing ring (30) being provided at the opening edge of the shell body (11) in order to secure the fitted ball head (22) and the internal diameter of the securing ring (30) being smaller than the diameter of the ball head (22), **characterised in that** the securing ring (30) comprises a circumferential groove (41) on its outside.

2. A prosthetic joint socket according to claim 1, **characterised in that** the securing ring (30) is arranged in a circumferential recess (35).

3. A prosthetic joint socket according to claim 1 or 2, **characterised in that** the securing ring (30) comprises a circumferential web (38) on its outside, which engages with a circumferential groove (37) on the inside of the shell body (11).

4. A prosthetic joint socket according to one of claims 1 to 3, **characterised in that** the securing ring (30) is made of a material which has lower hardness than the ball head material, and is preferably made of polyethylene.

## Revendications

1. Cotyle prothétique destiné à recevoir une tête de rotule (22), le cotyle étant formé d'un corps (11) formant coquille, simple, monobloc, le corps formant coquille étant en céramique, une bague de retenue (30) étant prévue au niveau du bord ouvert du corps formant coquille pour tenir la tête de rotule (22) insérée dans ledit corps, et le diamètre intérieur de la bague de retenue (30) étant inférieur au diamètre extérieur de la tête de rotule (22), **caractérisé en ce que** la bague de retenue (30), sur sa surface extérieure, comporte une gorge circulaire (41).

2. Cotyle prothétique selon la revendication 1, **caractérisé en ce que** la bague de retenue (30) est disposée dans un évidement (35) circulaire.

3. Cotyle prothétique selon la revendication 1 ou 2, **caractérisé en ce que** la bague de retenue (30), sur sa surface extérieure, comporte une nervure (38) circulaire qui s'engage dans une gorge (37) circulaire dans la surface intérieure du corps (11) formant coquille.

4. Cotyle prothétique selon une des revendications 1 à 3, **caractérisé en ce que** la bague de retenue (30) est en un matériau qui présente une dureté plus faible que le matériau de la tête de rotule et, de préférence en polyéthylène.
